# EUROPÄISCHE PATENTSCHRIFT

(11) **EP 3 795 670 B1**
(45) Veröffentlichungstag und Bekanntmachung des Hinweises auf die Patenterteilung: **23.07.2025**
(21) Anmeldenummer: 20196256.0
(22) Anmeldetag: 15.09.2020
(51) Int. Cl.: C12M 1/00, C12M 1/34, G01L 15/00, G01L 19/06, G01L 21/00, G01M 3/02, G01L 19/00, G01L 19/04

(54) **DRUCKMESS-EINWEGARTIKEL SOWIE DRUCKMESSSYSTEM MIT DERARTIGEM DRUCKMESS-EINWEGARTIKEL**
PRESSURE MEASURING DISPOSABLE ITEM AND PRESSURE MEASURING SYSTEM COMPRISING SUCH A PRESSURE MEASURING DISPOSABLE ITEM
ARTICLE JETABLE DE MESURE DE LA PRESSION AINSI QUE SYSTÈME DE MESURE DE LA PRESSION DOTÉ D'UN TEL ARTICLE

(30) Priorität: 19.09.2019 DE 102019125272
(43) Veröffentlichungstag der Anmeldung: 24.03.2021
(73) Patentinhaber: Labom Mess- und Regeltechnik GmbH, 27795 Hude (DE)
(72) Erfinder: Köster, Thomas, 26125 Oldenburg (DE)
(74) Vertreter: Wasiljeff, Johannes M.B.

(56) Entgegenhaltungen:
- WO-A1-2018/118458
- DE-A1- 10 144 230
- DE-A1- 102006 056 592
- DE-A1- 102010 018 377
- DE-A1- 3 924 740
- DE-B3- 102017 121 158
- DE-T2- 69 919 631
- DE-U1- 202016 008 710
- US-A1- 2007 074 575
- US-A1- 2018 202 881

## Beschreibung

Die Erfindung betrifft einen Druckmess-Einwegartikel für den einmaligen Gebrauch gemäß dem Oberbegriff von Anspruch 1 sowie ein Druckmesssystem mit einem derartigen Druckmess-Einwegartikel und mit einem über eine lösbare Verbindung lösbar mit dem Druckmess-Einwegartikel verbindbaren Druckmessgerät gemäß Anspruch 9.

Die Erfindung befasst sich mit der Druckmessung bei Anwendungen mit besonders hohen hygienischen Anforderungen. Bei derartigen Anwendungen werden verstärkt Einweggerätschaften für den einmaligen Gebrauch eingesetzt. Derartige Anwendungen umfassen bspw. die Kultivierung von (tierischen) Zellkulturen in Bioreaktoren. Während bei herkömmlichen Produktionsanlagen Kulturgefäße aus Glas oder Edelstahl verwendet werden, werden bei derartigen Anwendungen verstärkt Einwegbioreaktoren eingesetzt. Hierbei werden vorsterilisierte Einwegbeutel eingesetzt.

Nach der Herstellung einer Charge werden bei herkömmlichen Systemen bei derartigen Anwendungen mit besonders hohen hygienischen Anforderungen alle Komponenten sehr aufwändig, mit einem hohen Verbrauch an Energie und Wasser und damit sehr kostenintensiv gereinigt. Beim Einsatz von Einweggerätschaften werden hingegen die verwendeten Gerätschaften entsorgt, wobei sie üblicherweise verbrannt werden. Dies setzt allerdings voraus, dass die verwendeten Gerätschaften brennbar sind. Es ist daher bei einem derartigen Konzept geboten, die verwendeten Gerätschaften aus nicht-brennbaren Materialien, wie Metall, herzustellen. Tatsächlich werden regelmäßig aber auch Elemente mit integrierten Kabeln der Verbrennung zugeführt. Das in den Kabeln enthaltene Metall verbleibt in den Verbrennungsresten und erschwert die Entsorgung der Verbrennungsreste.

Im vorliegenden Zusammenhang bedeutet daher der Begriff "einmaliger Gebrauch" den Gebrauch während eines solchen Herstellungsprozesses, bspw. die Herstellung einer Charge einer Zellkultur.

Im Rahmen des Einsatzes derartiger Einweggerätschaften ist insbesondere vorgesehen, alle vom Prozessmedium (bspw. den Zellkulturen) berührten Oberflächen als Einwegartikel vorzusehen. Da diese Einwegartikel nach dem Gebrauch entsorgt werden, werden sie auch als Wegwerf-Artikel bezeichnet. Diese Einwegartikel werden daher typischerweise aus Kunststoffen gefertigt. Bekannt sind daher Schläuche, Beutel, etc., aus Kunststoffen.

Aufgrund der hohen hygienischen Anforderungen werden derartige Einwegartikel nach ihrer Herstellung und vor ihrer Verwendung gammasterilisiert, d.h. Gammastrahlung ausgesetzt, so dass dieser Artikel nach dieser Gammastrahlenbehandlung steril sind.

Nach jeder Produktionscharge, d.h. nach jedem Gebrauch werden derartige Einwegartikel entsorgt und in der Produktionsanlage werden neue Einwegartikel montiert.

Die Druckmessung bei derartigen auf Einweggerätschaften basierenden Produktionsanlagen mit besonders hohen hygienischen Anforderungen ist eine große Herausforderung, da einerseits für eine präzise Druckmessung möglichst ein direkter Kontakt mit dem Prozessmedium wünschenswert erscheint. Andererseits ist aber ein direkter Kontakt des Prozessmediums mit einem hochpräzisen Druckmesssystem nachteilig, da nach der Herstellung einer Charge das Druckmesssystem entweder ebenfalls entsorgt oder aufwändig gereinigt werden muss. Beides ist aber mit hohen Kosten verbunden.

Im Stand der Technik wurden daher die beiden folgenden Lösungsansätze verfolgt:
Ein erster Lösungsansatz besteht darin, einen einfachen Drucksensor samt Kabel in einem Einwegadapter zum Wegwerfen zu integrieren. Dieser Lösungsansatz ist jedoch insofern nachteilig, als dieser Einwegadapter Metall und Kabel enthält, wodurch die Entsorgung signifikant erschwert wird. Darüber hinaus wird bei diesem Lösungsansatz typischerweise nur ein kostengünstiger einfacher Sensor eingesetzt, der nur eine geringe Genauigkeit der Druckmessung bereitstellen kann.

Ein zweiter Lösungsansatz besteht darin, einen Einwegadapter mit einer Kunststoffmembran bereitzustellen, mit dem ein wiederverwendbares, herkömmliches Druckmessgerät verbunden wird.

Ein solches herkömmliches Druckmessgerät weist üblicherweise einen Druckmittler auf. Druckmittler dienen üblicherweise zur Übertragung von Drücken aus Prozessanlagen an einen Messumformer, bspw. um einen Füllstand in einem Flüssigkeitsbehälter zu messen. Derartige Druckmittler weisen meist einen metallischen Membrankörper mit einem oft konzentrisch ausgebildeten Membranbett sowie eine das Membranbett überspannende, dünne Membran auf. Der Zwischenraum zwischen Membranbett und Membran ist mit einer Druckübertragungsflüssigkeit gefüllt, die auch einen den Membrankörper durchsetzenden Kanal füllt, der in einem Hohlraum endet, in dem sich der eigentliche Druckaufnehmer befindet. Während üblicherweise die eine Oberfläche der Membran von dem Messmedium beaufschlagt wird, wirkt die gegenüberliegende Membranoberfläche auf die Druckübertragungsflüssigkeit. So wird der zu messende Druck vom Messmedium auf den Druckmessaufnehmer übertragen.

WO 2018/118458 offenbart den Anschluss eines wiederverwendbaren Druckmessgeräts an einen Einweg-Bio-Zellkulturreaktor, welches über eine flexible Membran in Wirkverbindung steht.

Gemäß dem zweiten Lösungsansatz wird jedoch die herkömmlicherweise mit dem Messmedium beaufschlagte Oberfläche mit dem Einwegadapter in Verbindung gebracht. Der Einwegadapter dient damit dazu, das Druckmessgerät an die Produktionsanlage anzuschließen, so dass das Druckmessgerät in der Lage ist, den Druck im Prozessmedium zu messen, ohne mit dem Prozessmedium in Kontakt zu gelangen.

Jedoch ist auch dieser zweite Lösungsansatz nachteilig, da zunächst die Kunststoffmembran mit der Membran eines Druckmittlers in Verbindung gebracht werden muss, wodurch ein Doppelmembransystem gebildet wird. Der Druck muss daher zunächst an die Kunststoffmembran und sodann weiter an die Druckmittlermembran weitergegeben werden, bevor er vom Druckmessgerät gemessen werden kann. Hieraus ergibt sich allerdings eine geringe Genauigkeit, die insbesondere bei kleinen Drücken zu ungenauen Messergebnissen führt.

Ein weiterer Nachteil dieses zweiten Lösungsansatzes besteht darin, dass ein Unterdruck im Prozessmedium praktisch nicht gemessen werden kann, da die Kunststoffmembran des Einwegadapters und die Druckmittlermembran nicht verbunden sind. Zwischen der Grundstoffmembran und der Druckmittlermembran wird sich daher im Falle eines Unterdrucks im Prozessmedium ein Zwischenraum bilden, der zu einer mangelhaften Druckübertragung und daher signifikanten Fehlmessungen führt.

Der Erfindung liegt daher die Aufgabe zu Grunde, die Druckmessung bei Anwendungen mit hohen hygienischen Anforderungen zu verbessern.

Die Erfindung löst diese Aufgabe mit den Merkmalen eines Druckmess-Einwegartikel gemäß Anspruch 1 sowie mit einem Druckmesssystem gemäß Anspruch 9.

Der erfindungsgemäße Druckmess-Einwegartikel für den einmaligen Gebrauch umfasst einen wenigstens einen Prozessanschluss aufweisenden Grundkörper, der eine Membran mit zwei Seiten aufweist, deren erste Seite vorgesehen ist, um mit dem Prozessmedium in Berührung zu gelangen. Dabei ist in den Druckmess-Einwegartikel ein mit Hydraulikflüssigkeit gefüllter Druckmittler derart integriert, dass die zweite Seite der Membran mit der Hydraulikflüssigkeit in Berührung steht, so dass diese Membran die Druckmittlermembran bildet.

Der Druckmittler weist dabei einen die Druckmittlerflüssigkeit führenden Kanal mit einem äußeren Ende auf, das einen Anschluss für ein lösbar mit dem Druckmittler verbindbares wiederzuverwendendes Druckmessgerät aufweist.

Das erfindungsgemäße Druckmesssystem umfasst einen derartigen Druckmess-Einwegartikel und ein über eine lösbare Verbindung lösbar mit dem Druckmess-Einwegartikel verbindbares mit Hydraulikflüssigkeit gefülltes Druckmessgerät, wobei die lösbare Verbindung eine Bajonettverbindung, eine Klemmverbindung und/oder Schraubverbindung umfasst. Eine derartige Verbindung erlaubt es dem Anwender den Druckmess-Einwegartikel auf einfache Weise und schnell vom Druckmessgerät zu trennen und einen neuen Druckmess-Einwegartikel zu montieren, so dass ein Wechsel des Druckmess-Einwegartikels nach Vollendung einer Chargenherstellung ebenfalls einfach und schnell erfolgen kann.

Die Erfindung schlägt somit ein Druckmesssystem vor, das ein wiederverwendbares Druckmessgerät - das einen Drucksensor, Elektronik und ein Gehäuse aufweist - und einen mit dem Druckmessgerät lösbar verbundenen Einweg-Druckmittler umfasst.

Dank der Integration des Druckmittlers in den Druckmess-Einwegartikel lässt sich der Druckmittler mit geringen Kosten herstellen und sterilisieren, wobei er aber zugleich den hohen Anforderungen an Einwegartikel bezüglich deren Entsorgbarkeit genügt.

Erfindungsgemäß ist der Druckmittler ebenso wie das Druckmessgerät jeweils separat mit Hydraulikflüssigkeit gefüllt. Bevorzugt geschieht diese Befüllung mit Hydraulikflüssigkeit bei der Herstellung des Druckmittlers und des Druckmessgeräts.

Die lösbare Verbindung ist dabei derart gestaltet, dass nach dem Verbinden des Druckmesssystems mit dem Druckmittler und damit mit dem Druckmess-Einwegartikel durch den Anwender ein druckdichter, vollständig flüssigkeitsgefüllter Raum entsteht, der eine hochqualitative Übertragung eines Druckes im Prozessmedium auf den Drucksensor im Druckmessgerät gestattet.

Das Druckmessgerät ist dabei derart ausgebildet, dass nach jedem Wechsel des Druckmittlers durch den Anwender erneut ein druckdichter, vollständig flüssigkeitsgefüllter Raum entsteht.

Die Erfindung ermöglicht somit auf vorteilhafte Weise eine kostengünstige Herstellung und Entsorgung der bei Anwendungen mit hohen hygienischen Anforderungen verwendeten Druckmess-Einwegartikel, da der Einwegartikel weder den Sensor, noch Kabel oder Stecker enthält. Der Druckmess-Einwegartikel kann daher vollständig aus einem entsorgbaren Material, wie Kunststoffen, gebildet sein.

Die Erfindung ermöglicht auf vorteilhafte Weise trotzdem eine hohe Genauigkeit der Druckmessung, die herkömmlichen Druckmittlersystemen nicht nachsteht, da ein wiederverwendbares Messgerät mit hoher Genauigkeit verwendet werden kann und folglich die Druckmessgenauigkeit nur von diesem wiederverwendbaren Messgerät abhängt.

Die Erfindung ermöglicht ferner auch Unterdrücke im Prozessmedium mit hoher Genauigkeit zu messen, da sie die Bildung eines Doppelmembransystem vermeidet und lediglich eine einzige Membran zur Druckübermittlung vom Prozessmedium zum Drucksensor erfordert.

Gemäß einer Weiterbildung der Erfindung ist der Grundkörper rohrartig und der Druckmittler als Rohrdruckmittler mit rohrförmiger Druckmittlermembran ausgebildet. Vorzugsweise wird dabei eine rohrförmige Folie in den im Inneren rohrförmigen Grundkörper eingeschweißt oder eingeklebt. Eine derartige Ausgestaltung ist vorteilhaft, da sie einerseits eine große Oberfläche der Druckmittlermembran bildet, ohne andererseits eine große Bauform des Druckmess-Einwegartikels zu erfordern. Eine derartige Ausbildung ermöglicht daher eine hohe Druckmessgenauigkeit. Zudem lässt sich eine derartige Ausbildung einfach in einer vorhandenen Prozessleitung montieren.

Eine Weiterbildung der Erfindung sieht vor, dass der Grundkörper ein Gehäuse mit einem oder mehreren Rohrenden zum Verbinden mit dem Prozessmedium bildet, in das die Druckmittlermembran als flache Membran integriert ist. Eine derartige Ausbildung erleichtert die Bildung der Membran und lässt sich daher einfach und kostengünstig herstellen.

Gemäß einer Weiterbildung der Erfindung ist der Druckmittler in einen Einwegbeutel, insbesondere in die Wand eines solchen Einwegbeutel, integriert, der als Kulturgefäß eines Einwegbioreaktors vorgesehen ist. Eine derartige Ausbildung stellt eine kostengünstige Lösung in der Zellkulturtechnik dar.

Eine Weiterbildung der Erfindung sieht vor, dass der Grundkörper ein Spritzgussteil aus Kunststoff ist, in das die Druckmittlermembran integriert ist. Da der Grundkörper auch den bzw. die Prozessanschlüsse aufweist, werden somit auch der bzw. die Prozessanschlüsse aus demselben Kunststoff spritzgegossen. Dies erlaubt eine besonders einfache und kostengünstige Herstellung des Druckmess- Einwegartikels.

Gemäß einer Weiterbildung der Erfindung ist die Druckmittlermembran in den Grundkörper eingeklebt, eingeklemmt und/oder eingeschweißt. Die Druckmittlermembran kann somit nachträglich am Grundkörper des Druckmess-Einwegartikels, d.h. nach Herstellung des Grundkörpers, angebracht werden. Diese Vorgehensweise erlaubt eine noch höhere Präzision bei der Herstellung der Druckmittlermembran und daher im Ergebnis eine noch höhere Messgenauigkeit als bei einer Herstellung der Druckmittlermembran zusammen mit dem Grundkörper.

Eine Weiterbildung der Erfindung sieht vor, dass die Druckmittlermembran konturiert ist. Dies erlaubt eine leichte Verformbarkeit der Druckmittlermembran, die wiederum vorteilhaft im Hinblick auf einen geringen Temperaturfehler, d.h. einen geringen temperaturbedingten Druckmessfehler ist und damit für eine hohe Genauigkeit der Druckmessung sorgt. Eine leicht verformbare Druckmittlermembran stellt sich im Wesentlichen als rückwirkungsfrei dar. D.h., dass eine Auslenkung der Membran im Bereich üblicher Druckmessungen keine signifikanten Gegenkräfte erzeugt, welche die Druckmessung nachteilig beeinflussen könnten.

Gemäß einer Weiterbildung der Erfindung ist die Druckmittlermembran aus einem besonders leicht verformbaren Material, wie bspw. Gummi gebildet. Eine derartige leichte Verformbarkeit der Membran führt dazu, dass eine Verformung der Membran zu keiner substanziellen Rückwirkung auf den zu messenden Druck führt. Durch diese Maßnahme lässt sich eine hohe Messgenauigkeit des Druckmesssystems erreichen.

Gemäß einer Weiterbildung der Erfindung umfasst die lösbare Verbindung zwischen dem Druckmess-Einwegartikel und dem Druckmessgerät eine hohle Nadel am Druckmessgerät, die bei hergestellter Verbindung zwischen Druckmittler und Druckmessgerät in den Kanal des Druckmittlers eindringt, insbesondere einsticht. Eine derartige Ausgestaltung ist vorteilhaft, da eine hohle Nadel sehr dünn ausgebildet werden kann, so dass die Hydraulikflüssigkeit bereits aufgrund ihrer Oberflächenspannung auch bei einem offenen Ausgang der Nadel nicht austreten kann. Daher ist eine Verbindung der Hydraulikflüssigkeit des Druckmessgeräts einerseits und des Druckmittlers andererseits (im Wesentlichen) ohne Einschluss von Luft möglich. Dies gewährleistet eine hohe Genauigkeit der Messergebnisse.

Gemäß einer Weiterbildung der Erfindung weist die hohle Nadel eine schräge Spitze auf. Eine derartige Ausgestaltung erleichtert das Durchstechen einer Versiegelung des die Hydraulikflüssigkeit im Druckmittler führenden Kanals an dessen äußerem Ende. Damit können auch ungeübte Anwender problemlos eine fehlerfreie Verbindung zwischen dem Druckmessgerät und einem ausgetauschten Druckmess-Einwegartikel herstellen.

Eine Weiterbildung der Erfindung sieht vor, dass die Hydraulikflüssigkeit im Druckmessgerät sowie im Druckmittler des Druckmess-Einwegartikels Wasser, insbesondere sterilisiertes und/oder destilliert Wasser, ist. Die Verwendung von Wasser als Hydraulikmedium ist vorteilhaft, da selbst im unwahrscheinlichen Fall eines Bruchs der Druckmittlermembran eine Kontamination des Prozessmediums bzw. der mit dem Prozess herzustellenden Charge - bspw. durch sonst üblicherweise verwendete Öle - vermieden wird.

Weiterbildungen der Erfindung ergeben sich aus den Ansprüchen, der Beschreibung und den Zeichnungen. Die vorgenannten Vorteile von Merkmalen und von Kombinationen mehrerer Merkmale sind beispielhaft und können alternativ oder kumulativ zur Wirkung kommen, ohne dass die Vorteile zwingend von erfindungsgemäßen Ausführungsformen erzielt werden müssen. Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausgestaltungen der Erfindung oder von Merkmalen unterschiedlicher Ansprüche ist ebenfalls abweichend von den gewählten Rückbeziehungen der Ansprüche möglich und wird hiermit vorgeschlagen. Dies betrifft auch solche Merkmale, die in separaten Zeichnungen dargestellt sind oder bei deren Beschreibung genannt werden. Diese Merkmale können auch mit Merkmalen verschiedener Ansprüche kombiniert werden. Ebenso können in Ansprüchen aufgeführte Merkmale für weitere Ausführungen der Erfindung entfallen.

In der Zeichnung zeigen:
- Fig. 1: ein erstes Ausführungsbeispiel eines erfindungsgemäßen Druckmesssystems mit einem erfindungsgemäßen Druckmess-Einwegartikel mit darauf montiertem wiederverwendbaren Druckmessgerät und
- Fig. 2: ein zweites Ausführungsbeispiel eines erfindungsgemäßen Druckmesssystems mit einem erfindungsgemäßen Druckmess-Einwegartikel mit darauf montiertem wiederverwendbaren Druckmessgerät.

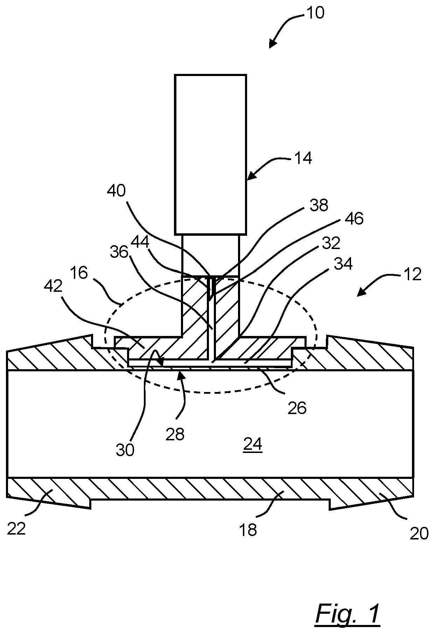

Fig. 1 zeigt ein erstes Ausführungsbeispiel eines erfindungsgemäßen Druckmesssystems 10, welches einen in einer Schnittansicht und daher schraffiert dargestellten erfindungsgemäßen Druckmess-Einwegartikel 12 aufweist. Ferner weist das Druckmesssystem 10 ein Druckmessgerät 14 auf, welches nicht schraffiert dargestellt ist.

Der Druckmess-Einwegartikel 12 weist einen Bereich auf, der einen Druckmittler 16 bildet. Ferner weist der Druckmess-Einwegartikel 12 einen Grundkörper 18 auf, der ein oder mehrere Prozessanschlüsse 20, 22 aufweist, die im dargestellten Ausführungsbeispiel als Schlauchanschlüsse ausgebildet sind.

Im Inneren des Grundkörpers 18 befindet sich während des Gebrauchs das Prozessmedium 24. Es kann bspw. mittels der Prozessanschlüsse 20, 22 durch den Druckmess-Einwegartikel 12 geführt werden. Sofern nur ein Prozessanschluss vorhanden oder aktiv ist, kann das Prozessmedium 24 über diesen Prozessanschluss zugeführt werden.

Im Inneren des Grundkörpers 18 ist eine nachfolgend als Druckmittlermembran 26 bezeichnete Membran des Druckmittlers 16 vorgesehenen. In dem Ausführungsbeispiel gemäß Fig. 1 ist diese Membran im Wesentlichen flach ausgebildet. Die Druckmittlermembran 26 ist daher im dargestellten Ausführungsbeispiel nicht etwa gewölbt, sondern nach Art einer Scheibe ausgebildet.

Die Druckmittlermembran 26 weist zwei Seiten auf, nämlich eine erste Seite 28, die in der Darstellung gemäß Fig. 1 nach unten gewandt ist. Diese erste Seite 28 gelangt daher mit dem Prozessmedium 24 in Berührung. Eine der ersten Seite 28 gegenüberliegende zweite Seite 30 der Druckmittlermembran 26 gelangt mit einer Hydraulikflüssigkeit 32 in Berührung, die in einem Innenraum 34 des Druckmittlers 16 enthalten ist. Die Hydraulikflüssigkeit 32 füllt diesen Innenraum 34 vollständig aus. Der Innenraum 34 umfasst einen ebenfalls Hydraulikflüssigkeit 32 führenden Kanal 36, der ein äußeres Ende 38 aufweist, das einen Anschluss 40 für das Druckmessgerät 14 umfasst. Das Druckmessgerät 14 ist über diesen Anschluss 40 lösbar mit dem Druckmess-Einwegartikel 12 verbunden. D.h., das Druckmessgerät 14 kann vom Anwender vom Druckmess-Einwegartikel 12 getrennt werden, um anschließend auf einen neuen Druckmess-Einwegartikel 12 montiert zu werden. Das Druckmessgerät 14 ist daher wiederverwendbar, während der Druckmess-Einwegartikel 12 als Wegwerf-Artikel vorgesehen ist, der nach dem einmaligen Gebrauch entsorgt wird.

Im dargestellten Ausführungsbeispiel ist der Grundkörper 18 allenfalls teilweise rohrförmig, insbesondere im Bereich der Prozessanschlüsse 20, 22. Im Bereich des Druckmittlers 16 weicht jedoch die innere Kontur des Grundkörpers 18 von der Rohrform ab, da - wie oben ausgeführt - in diesem Bereich die flach ausgebildete Druckmittlermembran 26 angeordnet ist. Im Bereich des Druckmittlers 16 kann daher der Grundkörper 18 eine quaderförmige oder zylinderförmige Kontur aufweisen.

Im in Fig. 1 dargestellten Ausführungsbeispiel ist der Druckmittler 16 neben der Druckmittlermembran 26 durch ein weiteres Teil, nämlich den Druckmittlerhauptkörper 42 gebildet. Dieser Druckmittlerhauptkörper 42 ist mit dem Grundkörper 18 verbunden. Bspw. sind der Druckmittlerhauptkörper 42 und der Grundkörper 18 miteinander verklebt, verschweißt, verschraubt oder mittels einer Klemmverbindung miteinander verbunden. Der Druckmittlerhauptkörper 42 und der Grundkörper 18 können aber auch einstückig hergestellt sein, bspw. im Rahmen eines 3-D-Druckverfahrens.

Vorteilhafterweise ist der Grundkörper 18 als Spritzgussteil aus Kunststoff hergestellt, in das die Druckmittlermembran 26 integriert ist.

Die Druckmittlermembran 26 kann - auch wenn dies aus Vereinfachungsgründen in Fig. 1 nicht dargestellt ist - konturiert, bspw. als Wellenmembran oder als Membran mit Sicken, ausgebildet sein, um vom unter dem Prozessdruck stehenden Prozessmedium 24 verformt werden zu können, ohne dass die Verformung signifikanten Einfluss auf den gemessenen Druck hat.

Über die Druckmittlermembran 26 und die sich im Innenraum 34 des Druckmittlers 16 befindenden Hydraulikflüssigkeit 32 wird der Druck über den Kanal 36 und den Anschluss 40 in das Druckmessgerät 14 übertragen und dort von einem nicht zeichnerisch dargestellten Drucksensor gemessen.

Das Druckmessgerät 14 ist zu diesem Zweck ebenfalls mit Hydraulikflüssigkeit gefüllt. Bei der Verbindung des Druckmessgeräts 14 mit dem Druckmess-Einwegartikel 12 erfolgt auch eine hydraulische Verbindung, wobei die Hydraulikflüssigkeit des Druckmessgeräts 14 mit der Hydraulikflüssigkeit 32 im Innenraum 34 des Druckmittlers 16 ohne Lufteinschlüsse in Verbindung gelangt. Zu diesem Zweck weist das Druckmessgerät 14 eine hohle Nadel 44 auf, die eine Fortsetzung des Kanals 36 in das Druckmessgerät 14 bewirkt.

Die Nadel 44 weist vorteilhafterweise eine schräge Spitze 46 auf, die geeignet ist, um in den Kanal 36 einzustechen, insbesondere wenn das äußere Ende 38 des Kanals 36 noch versiegelt ist.

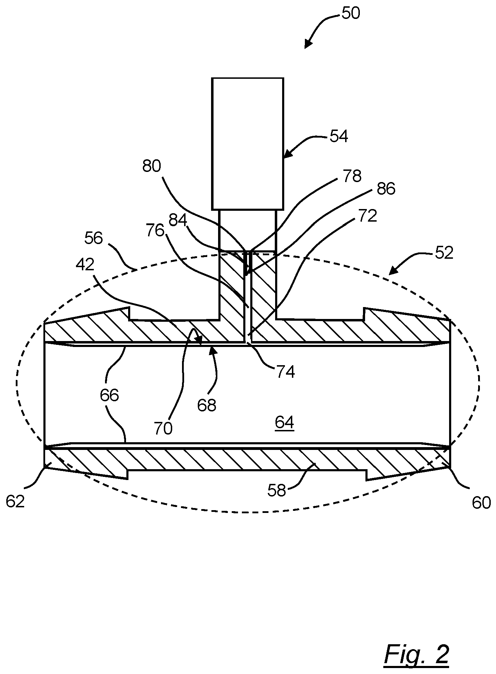

Fig. 2 zeigt ein zweites Ausführungsbeispiel eines erfindungsgemäßen Druckmesssystems 50, welches einen in einer Schnittansicht und daher schraffiert dargestellten erfindungsgemäßen Druckmess-Einwegartikel 52 aufweist. Ferner weist das Druckmesssystem 50 ein Druckmessgerät 54 auf, welches nicht schraffiert dargestellt ist und baugleich zu dem in Fig. 1 dargestellten Druckmessgerät 14 ausgebildet sein kann.

Der Druckmess-Einwegartikel 52 weist wiederum einen Bereich auf, der einen Druckmittler 56 bildet, der sich in der dargestellten Ausführungsform im Wesentlichen über die Länge eines Grundkörpers 58 ausdehnt. Dieser Grundkörper 58 weist ebenfalls einen oder mehrere Prozessanschlüsse 60, 62 auf, die im in Fig. 2 dargestellten Ausführungsbeispiel wiederum als Schlauchanschlüsse ausgebildet sind.

Im Inneren des Grundkörpers 58 befindet sich während des Gebrauchs das Prozessmedium 24. Ferner befindet sich im Inneren des Grundkörpers 58 eine rohrförmige Druckmittlermembran 66, die vorzugsweise als rohrförmige Folie ausgebildet und mit dem Grundkörper 58 verschweißt oder verklebt ist.

Auch die Druckmittlermembran 66 weist zwei Seiten auf, nämlich eine erste Seite 68, die dem Prozessmedium 64 zugewandt ist. Eine der ersten Seite 68 gegenüberliegende zweite Seite 70 der Druckmittlermembran 66 gelangt mit einer Hydraulikflüssigkeit 72 in Berührung, die in einem Innenraum 74 des Druckmittlers 56 enthalten ist. Die Hydraulikflüssigkeit 72 füllt diesen Innenraum 74 vollständig aus. Der Innenraum 74 umfasst wiederum einen Hydraulikflüssigkeit 72 führenden Kanal 76, der ein äußeres Ende 78 aufweist, das einen Anschluss 80 für das Druckmessgerät 54 umfasst.

Das Druckmessgerät 54 ist über diesen Anschluss 80 lösbar mit dem Druckmess-Einwegartikel 52 verbunden. Daher kann das Druckmessgerät 54 vom Anwender von dem Druckmess-Einwegartikel 52 getrennt werden, um anschließend auf einen neuen Druckmess-Einwegartikel 52 montiert zu werden. Daher ist auch das Druckmessgerät 54 wiederverwendbar, während der Druckmess-Einwegartikel 52 wiederum als Wegwerf-Artikel vorgesehen ist, der nach dem einmaligen Gebrauch entsorgt wird.

Im Ausführungsbeispiel gemäß Fig. 2 ist der Grundkörper 58 rohrartig und daher der Druckmittler 56 als Rohrdruckmittler mit rohrförmiger Druckmittlermembran 66 ausgebildet.

Vorteilhafterweise ist der Grundkörper 58 wiederum als ein Spritzgussteil aus Kunststoff hergestellt, während die Druckmittlermembran 66 aus einer Kunststofffolie gebildet ist.

Die Druckmittlermembran 66 kann - auch wenn dies wiederum aus Vereinfachungsgründen in Fig. 2 nicht dargestellt ist - konturiert ausgebildet sein, um vom unter dem Prozessdruck stehenden Prozessmedium 64 leichter verformt werden zu können, ohne dass die Verformung signifikanten Einfluss auf den gemessenen Druck hat.

Der über die Druckmittlermembran 66 und die sich im Innenraum 74 des Druckmittlers 56 befindenden Hydraulikflüssigkeit 72 wird der Druck über den Kanal 76 und den Anschluss 80 in das Druckmessgerät 54 übertragen und dort von einem nicht zeichnerisch dargestellten Drucksensor gemessen.

Entsprechend dem in Fig. 1 dargestellten Ausführungsbeispiel ist das Druckmessgerät 54 zu diesem Zweck ebenfalls mit Hydraulikflüssigkeit gefüllt. Bei einer Verbindung des Druckmessgeräts 54 mit dem Druckmess-Einwegartikel 52 erfolgt eine hydraulische Verbindung, wobei die Hydraulikflüssigkeit des Druckmessgeräts 54 mit der Hydraulikflüssigkeit 72 im Innenraum 74 des Druckmittlers 56 in Verbindung gelangt. Zu diesem Zweck weist das Druckmessgerät 54 wiederum eine hohle Nadel 84 auf, die eine Fortsetzung des Kanals 76 in das Druckmessgerät 54 bewirkt. Vorteilhafterweise weist auch diese Nadel 84 eine schräge Spitze 86 auf, die geeignet ist, um in den Kanal 76 einzustechen, insbesondere wenn das äußere Ende 78 sich des Kanals 76 versiegelt ist.

In den dargestellten Ausführungsbeispielen ist der Druckmittler in ein Druckmess-Einwegartikel integriert, der primär zum Leiten des Prozessmediums vorgesehen ist. In anderen Ausführungsbeispielen kann der Druckmittler aber auch in andere Einwegartikel integriert sein, wie bspw. in einen Einwegbeutel, der als Kulturgefäß eines Einwegbioreaktors vorgesehen ist. In einem weiteren Ausführungsbeispiel kann der Druckmittler in einem Einweggefäß integriert sein, das bspw. als Tank vorgesehen ist.

Insgesamt ermöglicht die Erfindung die Druckmessung bei Anwendungen mit besonders hohen hygienischen Anforderungen zu verbessern, indem ein hochwertiges wiederzuverwendendes Druckmessgerät mit einem als Wegwerf-Artikel vorgesehenen Druckmittler kombiniert wird.

Vorliegend werden folgende Bezugszeichen verwendet:
- 10: Druckmesssystem
- 12: Druckmess-Einwegartikel
- 14: Druckmessgerät
- 16: Druckmittler
- 18: Grundkörper
- 20: Prozessanschluss
- 22: Prozessanschluss
- 24: Prozessmedium
- 26: Druckmittlermembran
- 28: erste Seite der Druckmittlermembran
- 30: zweite Seite der Druckmittlermembran
- 32: Hydraulikflüssigkeit
- 34: Innenraum des Druckmittlers
- 36: Kanal
- 38: äußeres Ende des Kanals
- 40: Anschluss des Druckmessgeräts
- 42: Druckmittlerhauptkörper
- 44: hohle Nadel
- 46: schräge Spitze
- 50: Druckmesssystem
- 52: Druckmess-Einwegartikel
- 54: Druckmessgerät
- 56: Druckmittler
- 58: Grundkörper
- 60: Prozessanschluss
- 62: Prozessanschluss
- 64: Prozessmedium
- 66: Druckmittlermembran
- 68: erste Seite der Druckmittlermembran 66
- 70: zweite Seite der Druckmittlermembran 66
- 72: Hydraulikflüssigkeit
- 74: Innenraum des Druckmittlers 56
- 76: Kanal
- 78: äußeres Ende des Kanals 76
- 80: Anschluss für Druckmessgerät 54
- 84: hohle Nadel des Druckmessgeräts 54
- 86: schräge Spitze

## Patentansprüche

1. Druckmess-Einwegartikel für den einmaligen Gebrauch mit einem wenigstens einen Prozessanschluss (20, 22; 60, 62) aufweisenden Grundkörper (18; 58), der eine Membran (26; 66) mit zwei Seiten (28, 30; 68, 70) aufweist, deren erste Seite (28; 68) vorgesehen ist, um mit einem Prozessmedium (24; 64) in Berührung zu gelangen,
**dadurch gekennzeichnet, dass**
in den Druckmess-Einwegartikel (12; 52) ein mit Hydraulikflüssigkeit (32; 72) gefüllter Druckmittler (16; 56) derart integriert ist, dass die zweite Seite (30; 70) der Membran (26; 66) mit der Hydraulikflüssigkeit (32; 72) in Berührung steht, so dass die Membran (26; 66) die Druckmittlermembran bildet,
wobei der Druckmittler (16; 56) einen die Hydraulikflüssigkeit (32; 72) führenden Kanal (36; 76) mit einem äußeren Ende (38; 78) aufweist, das einen Anschluss (40; 80) für ein lösbar mit dem Druckmittler (16; 56) verbindbares wiederzuverwendendes Druckmessgerät (14; 54) aufweist.

2. Druckmess-Einwegartikel nach Anspruch 1,
**dadurch gekennzeichnet, dass**
der Grundkörper (58) rohrartig und der Druckmittler (56) als Rohrdruckmittler mit rohrförmiger Druckmittlermembran (66) ausgebildet ist.

3. Druckmess-Einwegartikel nach Anspruch 1,
**dadurch gekennzeichnet, dass**
der Grundkörper (18) ein Gehäuse mit einem oder mehreren Rohrenden zum Verbinden mit dem Prozessmedium (24) bildet, in das die Druckmittlermembran (26) als flache Membran integriert ist.

4. Druckmess-Einwegartikel nach Anspruch 1,
**dadurch gekennzeichnet, dass**
der Druckmittler in einen Einwegbeutel integriert ist, der als Kulturgefäß eines Einwegbioreaktors vorgesehen ist.

5. Druckmess-Einwegartikel nach einem der vorhergehenden Ansprüche,
**dadurch gekennzeichnet, dass**
die Druckmittlermembran (26; 66) in den Grundkörper (18; 58) eingeklebt, eingeklemmt und/oder eingeschweißt ist.

6. Druckmess-Einwegartikel nach einem der vorhergehenden Ansprüche,
**dadurch gekennzeichnet, dass**
die Druckmittlermembran (26; 66) konturiert ist.

7. Druckmess-Einwegartikel nach einem der vorhergehenden Ansprüche,
**dadurch gekennzeichnet, dass**
die Druckmittlermembran (26; 66) aus einem besonders leicht verformbaren Material, wie bspw. Gummi, gebildet ist.

8. Druckmess-Einwegartikel nach einem der Ansprüche 1 bis 6,
**dadurch gekennzeichnet, dass**
der Grundkörper (18; 58) ein Spritzgussteil aus Kunststoff ist, in das die Druckmittlermembran (26; 66) integriert ist.

9. Druckmesssystem mit einem Druckmess-Einwegartikel (12; 52) nach einem der vorhergehenden Ansprüche und mit einem über eine lösbare Verbindung lösbar mit dem Druckmess-Einwegartikel (12; 52) verbindbaren mit Hydraulikflüssigkeit (32; 72) gefüllten Druckmessgerät (14; 54), wobei die lösbare Verbindung eine Bajonettverbindung, eine Klemmverbindung und/oder Schraubverbindung umfasst.

10. Druckmesssystem nach Anspruch 9,
wobei die lösbare Verbindung eine hohle Nadel (44; 84) am Druckmessgerät (14; 54) umfasst, die bei hergestellter Verbindung zwischen Druckmittler (16; 56) und Druckmessgerät (14; 54) in den Kanal (36; 76) des Druckmittlers (16; 56) eindringt, vorzugsweise einsticht.

11. Druckmesssystem nach Anspruch 10,
wobei die hohle Nadel (44; 84) eine schräge Spitze (46; 86) aufweist.

12. Druckmesssystem nach einem der Ansprüche 9 bis 11, wobei die Hydraulikflüssigkeit (32; 72) Wasser, bevorzugt sterilisiertes Wasser, ist.

13. Druckmess-Einwegartikel (12; 52) nach einem der Ansprüche 1 bis 8, wobei die Hydraulikflüssigkeit (32; 72) Wasser, bevorzugt sterilisiertes Wasser, ist.

## Claims

1. Pressure measuring disposable item for single use with a base body (18; 58) having at least one process connection (20, 22; 60, 62), which has a membrane (26; 66) with two sides (28, 30; 68, 70), the first side (28; 68) of which is intended to come into contact with a process medium (24; 64),
**characterized in that**
a diaphragm seal (16; 56) filled with hydraulic fluid (32; 72) is integrated into the pressure measuring disposable item (12; 52) in such a way that the second side (30; 70) of the membrane (26; 66) is in contact with the hydraulic fluid (32; 72), so that the membrane (26; 66) forms the diaphragm seal membrane,
wherein the diaphragm seal (16; 56) has a channel (36; 76) carrying the hydraulic fluid (32; 72) with an outer end (38; 78) which has a connection (40; 80) for a reusable pressure measuring device (14; 54) which can be detachably connected to the diaphragm seal (16; 56).

2. Pressure measuring disposable item according to claim 1,
**characterized in that**
the base body (58) is tubular and the diaphragm seal (56) is designed as a tubular diaphragm seal with a tubular diaphragm seal membrane (66).

3. Pressure measuring disposable item according to claim 1,
**characterized in that**
the base body (18) forms a housing with one or more tube ends for connection to the process medium (24), into which the diaphragm seal membrane (26) is integrated as a flat membrane.

4. Pressure measuring disposable item according to claim 1,
**characterized in that**
the diaphragm seal is integrated into a disposable bag which is provided as a culture vessel of a disposable bioreactor.

5. Pressure measuring disposable item according to one of the preceding claims,
**characterized in that**
the diaphragm seal membrane (26; 66) is glued, clamped and/or welded into the base body (18; 58).

6. Pressure measuring disposable item according to one of the preceding claims,
**characterized in that**
the diaphragm seal membrane (26; 66) is contoured.

7. Pressure measuring disposable item according to one of the preceding claims,
**characterized in that**
the diaphragm seal membrane (26; 66) is formed from a particularly easily deformable material, such as rubber.

8. Pressure measuring disposable item according to one of claims 1 to 6,
**characterized in that**
the base body (18; 58) is an injection-molded part made of plastic, into which the diaphragm seal membrane (26; 66) is integrated.

9. Pressure measuring system with a pressure measuring disposable item (12; 52) according to one of the preceding claims and with a pressure measuring device (14; 54) filled with hydraulic fluid (32; 72) which can be detachably connected to the pressure measuring disposable item (12; 52) via a detachable connection, wherein the detachable connection comprises a bayonet connection, a clamp connection and/or a screw connection.

10. Pressure measuring system according to claim 9,
wherein the detachable connection comprises a hollow needle (44; 84) on the pressure measuring device (14; 54) which, when the connection between the diaphragm seal (16; 56) and the pressure measuring device (14; 54) is established, penetrates, preferably punctures, the channel (36; 76) of the diaphragm seal (16; 56).

11. Pressure measuring system according to claim 10,
wherein the hollow needle (44; 84) has a slanted tip (46; 86).

12. Pressure measuring system according to one of claims 9 to 11,
wherein the hydraulic fluid (32; 72) is water, preferably sterilized water.

13. Pressure measuring disposable item (12; 52) according to one of claims 1 to 8,
wherein the hydraulic fluid (32; 72) is water, preferably sterilized water.

## Revendications

1. Article jetable de mesure de la pression pour l'usage unique avec au moins un corps de base (18, 58) comportant un raccord de processus (20, 22, 60, 62), qui comporte une membrane (26, 66) avec deux côtés (28, 30, 68, 70), dont un premier côté (28, 68) est prévu pour parvenir en contact avec un milieu de processus (24, 64)
**caractérisé en ce qu'**
un transmetteur de pression (16,56) rempli de liquide hydraulique (32, 72) est intégré dans l'article jetable de mesure de la pression (12, 52) de telle manière que le deuxième côté (30, 70) de la membrane (26, 66) se trouve en contact avec le liquide hydraulique (32, 72) de sorte que la membrane (26, 66) forme la membrane de transmetteur de pression,
sachant que le transmetteur de pression (16, 56) comporte un conduit (36, 76) guidant le liquide hydraulique (32, 72) avec une extrémité extérieure (38, 78), qui comporte un raccord (40, 80) pour un appareil de mesure de la pression (14, 54) à réutiliser pouvant est relié de façon amovible avec le transmetteur de pression (16, 56).

2. Article jetable de mesure de la pression selon la revendication 1,
**caractérisé en ce que**
le corps de base (58) est constitué de type tubulaire et le transmetteur de pression (56) sous la forme d'un transmetteur de pression tubulaire avec membrane de transmetteur de pression tubulaire (66).

3. Article jetable de mesure de la pression selon la revendication 1,
**caractérisé en ce que**
le corps de base (18) forme un boîtier avec une ou plusieurs extrémités tubulaires pour raccorder au milieu de processus (24) dans lequel est intégrée la membrane de transmetteur de pression (26) en tant que membrane plate.

4. Article jetable de mesure de la pression selon la revendication 1,
**caractérisé en ce que**
le transmetteur de pression est intégré dans un sac jetable, qui est prévu en tant que récipient de culture d'un bioréacteur jetable.

5. Article jetable de mesure de la pression selon au moins l'une quelconque des revendications précédentes,
**caractérisé en ce que**
la membrane du transmetteur de pression (26, 66) est collée, serrée et/ou soudée dans le corps de base (18, 58).

6. Article jetable de mesure de la pression selon l'une quelconque des revendications précédentes,
**caractérisé en ce que**
la membrane du transmetteur de pression (26, 66) est profilée.

7. Article jetable de mesure de la pression selon au moins l'une quelconque des revendications précédentes,
**caractérisé en ce que**
la membrane du transmetteur de pression (26, 66) est formée d'un matériau en particulier facilement déformable, comme par exemple du caoutchouc.

8. Article jetable de mesure de la pression selon l'une quelconque des revendications 1 à 6,
**caractérisé en ce que**
le corps de base (18, 58) est une pièce moulée par injection en matière plastique, dans laquelle est intégrée la membrane du transmetteur de pression (26, 66) .

9. Système de mesure de la pression avec un article jetable de mesure de la pression (12, 52) selon l'une quelconque des revendications précédentes et avec un appareil de mesure de la pression (14, 54) rempli de liquide hydraulique (32, 72) pouvant être relié de façon amovible par une liaison séparable à l'article jetable de mesure de la pression (12, 52), sachant que la liaison séparable comprend une liaison à baïonnette, une liaison par serrage et/ou une liaison par vissage.

10. Système de mesure de la pression selon la revendication 9,
sachant que la liaison séparable comprend une aiguille creuse (44, 84) sur l'appareil de mesure de la pression (14, 54), qui une fois la liaison réalisée entre le transmetteur de pression (16, 56) et l'appareil de mesure de la pression (14, 54) pénètre, de préférence s'enfonce, dans le conduit (36, 76) du transmetteur de pression (16, 56).

11. Système de mesure de la pression selon la revendication 10,
sachant que l'aiguille creuse (44, 84) comporte une point inclinée (46, 86).

12. Système de mesure de la pression selon au moins l'une quelconque des revendications 9 à 11, sachant que le liquide hydraulique (32, 72) est de l'eau de préférence de l'eau stérilisée.

13. Article jetable de mesure de la pression (12, 52) selon l'une quelconque des revendications 1 à 8, sachant que le liquide hydraulique (32, 72) est de l'eau de préférence de l'eau stérilisée.
